# DEMANDE DE BREVET EUROPEEN

(11) **EP 3 501 606 A1**
(43) Date de publication de la demande: **26.06.2019**
(21) Numéro de dépôt: 18215044.1
(22) Date de dépôt: 21.12.2018
(51) Int. Cl.: A61P 9/08, A61K 36/9068, A61K 36/16, A61K 36/254, A61K 36/258, A61K 36/54, A61K 47/36

(54) **ASSOCIATION D'UN ACTIF VASODILATATEUR À BASE DE PLANTE ET D'ACIDE HYALURONIQUE POUR AMÉLIORER LA SENSIBILITÉ TACTILE**

(30) Priorité: 21.12.2017 FR 1762846
(71) Demandeur: Laboratoires Chemineau, 37210 Vouvray (FR)
(72) Inventeur: SACRE, Morgan, 37210 VOUVRAY (FR); HUET, Grégoire, 75011 PARIS (FR); DESSABLES, Estelle, 37320 Esvres (FR); AGNUS, Benoît, 37110 Villedomer (FR)
(74) Mandataire: Cabinet Plasseraud

(57) **Abrégé**

La présente invention a pour objet un actif vasodilatateur choisi dans le groupe comprenant le ginseng brésilien (*Pfaffia glomerata*), le ginseng sibérien (*Eleutherococcus senticosus)*, le ginseng rouge de Corée (*Panax ginseng*), le ginseng américain (*Panax quinquefolius*), la maca (*Lepidium meyenii*), le guarana (*Paullinia cupana*), la caféine, la théine, le muira puama (*Ptychopetalum olacoides*), l'abricotier d'argent (*Ginkgo biloba*), le yohimbe (*Pausinystalia johimbe*), le tribulus *(Tribulus terrestris)*, le gingembre (*Zingiber officinale*), la cannelle (*Cinnamomum verum*), le céleri (*Apium graveolens*) et leurs mélanges, et de préférence le ginseng brésilien,
en association avec de l'acide hyaluronique et/ou ses sels,
et éventuellement avec un agent hydratant choisi dans le groupe comprenant des héparanes sulfates, du glycérol, un polysaccharide anionique (Biosaccharide gum-1), du xylitol, du fructose, du collagène, un ester triple de glycérol et des acides caprylique et caprique (nom INCI : caprilic/capric triglycérides), du caprylyl caprylate/caprate (nom INCI), de la nicotinamide, de l'Aloe vera (*Aloe barbadensis*) et leurs mélanges, et de préférence des héparanes sulfates,
pour une utilisation dans la prévention et/ou le traitement des troubles de la somesthésie, et plus particulièrement de l'hypoesthésie.

## Description

La présente invention a pour objet une association d'un actif vasodilatateur à base de plante, notamment de ginseng brésilien, et d'acide hyaluronique et/ou ses sels pour améliorer la sensibilité tactile, notamment chez des sujets âgés d'au moins 35 ans, et de préférence d'au moins 60 ans.

En 2035 avec l'augmentation de l'espérance de vie, la proportion des français de plus de 60 ans représentera 31% de la population contre 22% aujourd'hui. Le vieillissement est un problème de société, mais c'est aussi un marché économique. Ainsi, la pharmacologie et la cosmétique s'intéressent au vieillissement cutané, car la peau, à l'instar des autres organes, vieillit. Les premiers signes de vieillissement cutané apparaissent après 30 ans, lorsque la synthèse de collagène et d'élastine diminue. En conséquence, l'élasticité décroît.

Pour limiter les changements structuraux de la peau liés à l'âge, la pharmacologie et la cosmétique proposent un certain nombre de produits qui ont fait leur preuve. Ces traitements locaux visent quatre cibles potentielles : une barrière chimique et mécanique susceptible de préserver l'intégrité physique de l'épiderme, la préservation de la balance ionique de l'organisme, la promotion des défenses immunitaires et l'élimination des toxines, et enfin la protection contre les radiations solaires et les produits oxydants.

Classiquement, les études évaluent l'effet des produits cosmétiques sur l'architecture de la peau mais elles ne considèrent jamais les conséquences fonctionnelles d'un quelconque changement de la peau en termes de sensibilité tactile. L'élasticité moindre de la peau a pourtant des conséquences sensorielles. Nous perdons en sensibilité tactile car la transmission des contraintes mécaniques (vibrations et pressions) générées par l'exploration tactile diminue. Dès 30 ans, le seuil de détection aux pressions et vibrations augmente (1). Nous perdons alors 1% par an de capacité à discriminer deux pointes appliquées simultanément sur la peau, notamment aux extrémités distales (2). Cette déficience tactile en dehors de toute pathologie aura de plus en plus de retentissement sur la qualité de vie avec le développement des interfaces tactiles numériques (téléphones portables, tablettes, etc.).

Les Inventeurs de la présente invention ont à présent découvert de manière surprenante que l'association d'un actif vasodilatateur, comme par exemple le ginseng brésilien, et d'acide hyaluronique et/ou ses sels permettait d'augmenter de façon significative la capacité de discrimination tactile chez des sujets, et notamment des sujets âgés d'au moins 35 ans, de préférence d'au moins 55 ans, et encore plus préférentiellement d'au moins 60 ans.

La présente invention a ainsi pour objet un actif vasodilatateur choisi dans le groupe comprenant le ginseng brésilien (*Pfaffia glomerata*), le ginseng sibérien (*Eleutherococcus senticosus),* le ginseng rouge de Corée (*Panax ginseng*), le ginseng américain (*Panax quinquefolius*)*,* la maca (*Lepidium meyenii*)*,* le guarana (*Paullinia cupana*), la caféine, la théine, le muira puama (*Ptychopetalum olacoides*), l'abricotier d'argent *(Ginkgo biloba*), le yohimbe (*Pausinystalia johimbe*), le tribulus *(Tribulus terrestris)*, le gingembre *(Zingiber officinale),* la cannelle (*Cinnamomum verum*), le céleri (*Apium graveolens*) et leurs mélanges, et de préférence le ginseng brésilien,
en association avec de l'acide hyaluronique et/ou ses sels,
et éventuellement avec un agent hydratant choisi dans le groupe comprenant des héparanes sulfates, du glycérol, un polysaccharide anionique (Biosaccharide gum-1), du xylitol, du fructose, du collagène, un ester triple de glycérol et des acides caprylique et caprique (nom INCI: caprilic/capric triglycérides), du caprylyl caprylate/caprate (nom INCI), de la nicotinamide, de l'Aloe vera (*Aloe Barbadensis*) et leurs mélanges, et de préférence des héparanes sulfates,
pour une utilisation dans la prévention et/ou le traitement des troubles de la somesthésie, et plus particulièrement de l'hypoesthésie.

La somesthésie (dite aussi sensibilité du corps) est le principal système sensoriel de l'organisme humain. La somesthésie désigne un ensemble de différentes sensations (pression, chaleur, douleur...) qui proviennent de plusieurs régions du corps (peau, tendons, articulations, viscères...). Ces sensations sont élaborées à partir des informations fournies par de nombreux récepteurs sensitifs du système somatosensoriel, situés dans les tissus de l'organisme.

L'hypoesthésie peut être considérée comme un exemple particulier de trouble de la somesthésie. L'hypoesthésie désigne une diminution de la sensibilité du toucher (sensibilité tactile) face à des stimuli.

Le Ginseng brésilien (*Pfaffia glomerata*) présente des propriétés nutritionnelles et préventives qui se rapprochent de celles du ginseng dit « classique ». Le Ginseng brésilien est utilisé depuis des temps immémoriaux par les Indiens comme tonique général et anti-fatigue, mais aussi comme régulateur de l'appareil digestif. Au Brésil, le Pfaffia est connu pour améliorer l'oxygénation des cellules, stimuler l'appétit et la circulation, renforcer le système immunitaire, le système musculaire et la mémoire.

Le Pfaffia pousse sur les versants méridionaux des montagnes humides au milieu des forêts brésiliennes. Il appartient à la famille des Amaranthacées et possède une grosse racine tubéreuse, à partir de laquelle il est possible de recueillir une sève, par pression à froid.

Selon un mode de réalisation avantageux de l'invention, le Ginseng brésilien est plus particulièrement utilisé sous forme de jus de racine.

Selon un autre mode de réalisation avantageux de l'invention, l'acide hyaluronique est un mélange de deux acides hyaluroniques, de préférence sous forme de leurs sels, présentant respectivement un poids moléculaire (PM) allant de 100 à 300 KDa et allant de 1000 à 1400 KDa (kilodaltons).

A titre d'exemple d'un sel d'acide hyaluronique, on pourra citer l'hyaluronate de sodium qui est le sel sodique de l'acide hyaluronique.

De façon préférée, l'hyaluronate de sodium utilisé selon l'invention se trouve sous la forme d'un mélange de deux hyaluronates de sodium, d'un poids moléculaire (PM) respectif allant de 100 à 300 KDa et allant de 1000 à 1400 KDa.

Selon encore un mode de réalisation avantageux de l'invention, l'actif vasodilatateur en association avec de l'acide hyaluronique et/ou ses sels pour une utilisation telle que définie ci-dessus, est particulièrement approprié pour une utilisation chez des sujets âgés d'au moins 35 ans, de préférence d'au moins 55 ans, et plus préférentiellement encore d'au moins 60 ans.

En effet, comme expliqué précédemment, les premiers signes de vieillissement cutané apparaissent après 30 ans, lorsque la synthèse de collagène et d'élastine diminue. Les conséquences en sont ainsi une baisse d'élasticité de la peau mais aussi une baisse de sensibilité tactile.

Selon un autre mode de réalisation avantageux de l'invention, l'actif vasodilatateur en association avec de l'acide hyaluronique et/ou ses sels pour une utilisation telle que définie ci-dessus, est particulièrement approprié pour une application :
- sur les mains et/ou les pieds, et/ou
- sur les muqueuses buccales, anales et/ou vaginales.

L'invention a également pour objet une composition topique comprenant :
- un actif vasodilatateur choisi dans le groupe comprenant le ginseng brésilien (*Pfaffia glomerata*), le ginseng sibérien (*Eleutherococcus senticosus),* le ginseng rouge de Corée (*Panax ginseng*), le ginseng américain (*Panax quinquefolius*), la maca (*Lepidium meyenii*)*,* le guarana (*Paullinia cupana*), la caféine, la théine, le muira puama (*Ptychopetalum olacoides*), l'abricotier d'argent (*Ginkgo biloba*), le yohimbe (*Pausinystalia johimbe*), le tribulus *(Tribulus terrestris),* le gingembre (*Zingiber officinale*), la cannelle (*Cinnamomum verum*), le céleri (*Apium graveolens*) et leurs mélanges, et de préférence le ginseng brésilien,
- l'acide hyaluronique et/ou ses sels,
- éventuellement un agent hydratant choisi dans le groupe comprenant des héparanes sulfates, du glycérol, un polysaccharide anionique (Biosaccharide gum-1), du xylitol, du fructose, du collagène, un ester triple de glycérol et des acides caprylique et caprique (nom INCI: caprilic/capric triglycérides), du caprylyl caprylate/caprate (= nom INCI), de la nicotinamide, de l'Aloe vera (*Aloe barbadensis*) et leurs mélanges, et de préférence des héparanes sulfates,
- de l'eau,
- éventuellement un agent de conservation choisi dans le groupe comprenant le pentylène glycol, le sorbate de potassium, le phénoxyéthanol, l'éthyl héxyl la glycérine, le benzoate de sodium, le gluconolactone, le capryloyl glycine, l'octopirox, l'alcool phényléthylique et leurs mélanges,
pour une utilisation dans la prévention et/ou le traitement des troubles de la somesthésie, et plus particulièrement de l'hypoesthésie.

De façon préférée, la composition de l'invention pour une utilisation telle que définie ci-dessus est dépourvue de sel d'arginine de l'acide L-pyrrolidone carboxylique (L-PCA).

Selon un mode de réalisation, la composition topique de l'invention pour une utilisation telle que définie ci-dessus est constituée :
- d'un actif vasodilatateur choisi dans le groupe comprenant le ginseng brésilien (*Pfaffia glomerata*), le ginseng sibérien (*Eleutherococcus senticosus),* le ginseng rouge de Corée *(Panax ginseng*), le ginseng américain (*Panax quinquefolius*), la maca (*Lepidium meyenii*)*,* le guarana (*Paullinia cupana*), la caféine, la théine, le muira puama (*Ptychopetalum olacoides*), l'abricotier d'argent (*Ginkgo biloba*), le yohimbe (*Pausinystalia johimbe*), le tribulus *(Tribulus terrestris),* le gingembre (*Zingiber officinale*), la cannelle (*Cinnamomum verum*)*,* le céleri (*Apium graveolens*) et leurs mélanges, et de préférence le ginseng brésilien,
- d'acide hyaluronique et/ou ses sels, et
- d'eau.

Selon un autre mode de réalisation, la composition topique de l'invention pour une utilisation telle que définie ci-dessus est constituée :
- d'un actif vasodilatateur choisi dans le groupe comprenant le ginseng brésilien (*Pfaffia glomerata*), le ginseng sibérien (*Eleutherococcus senticosus),* le ginseng rouge de Corée (*Panax ginseng*), le ginseng américain (*Panax quinquefolius*), la maca (*Lepidium meyenii*), le guarana (*Paullinia cupana*), la caféine, la théine, le muira puama (*Ptychopetalum olacoides*), l'abricotier d'argent (*Ginkgo biloba*), le yohimbe (*Pausinystalia johimbe*), le tribulus *(Tribulus terrestris),* le gingembre (*Zingiber officinale*), la cannelle (*Cinnamomum verum*), le céleri (*Apium graveolens*) et leurs mélanges, et de préférence le ginseng brésilien,
- d'acide hyaluronique et/ou ses sels,
- d'un agent hydratant choisi dans le groupe comprenant des héparanes sulfates, du glycérol, un polysaccharide anionique (Biosaccharide gum-1), du xylitol, du fructose, du collagène, un ester triple de glycérol et des acides caprylique et caprique (nom INCI: caprilic/capric triglycérides), du caprylyl caprylate/caprate (= nom INCI), de la nicotinamide, de l'Aloe vera et leurs mélanges, et de préférence des héparanes sulfates,
- d'eau,
- d'un agent de conservation choisi dans le groupe comprenant le pentylène glycol, le sorbate de potassium, le phénoxyéthanol, l'éthyl héxyl la glycérine, le benzoate de sodium, le gluconolactone, le capryloyl glycine, l'octopirox, l'alcool phényléthylique et leurs mélanges.

La composition topique selon l'invention pourra encore comprendre un ou plusieurs excipients permettant d'obtenir la galénique recherchée pour la composition topique de l'invention.

Plus particulièrement, dans la composition topique pour une utilisation telle que définie ci-dessus :
- l'actif vasodilatateur est présent en une quantité allant de 1 à 10%, de préférence de 1,5 à 5%,
- l'acide hyaluronique et/ou ses sels est présent en une quantité allant de 0,01 à 1%, de préférence de 0,05 à 0,5%,
- l'agent hydratant est présent en une quantité allant de 3 à 30%, de préférence de 5 à 15%,
- l'eau est présente en une quantité allant de 60 à 90%, de préférence de 70 à 85%,
- l'agent de conservation est présent en une quantité allant de 0,1 à 5%, de préférence de 0 ,5 à 2,5%,
les pourcentages étant exprimés en poids par rapport au poids total de la composition.

La composition topique de l'invention pour une utilisation telle que définie ci-dessus est particulièrement appropriée pour une application sur les mains et/ou les pieds.

Selon un autre mode de réalisation, la composition topique de l'invention pour une utilisation telle que définie ci-dessus est particulièrement appropriée pour une application sur les sur les muqueuses buccales, anales et/ou vaginales.

Selon un mode de réalisation avantageux de l'invention, la composition topique pour une utilisation telle que définie ci-dessus, est particulièrement appropriée pour une utilisation chez des sujets âgés d'au moins 35 ans, de préférence d'au moins 55 ans, et plus préférentiellement encore d'au moins 60 ans.

En effet, l'application de la composition de l'invention telle que définie ci-dessus permet d'augmenter de façon significative la capacité de discrimination tactile chez des sujets, et notamment chez des sujets âgés d'au moins 35 ans, de préférence d'au moins 55 ans, et plus préférentiellement encore d'au moins 60 ans.
La figure 1 illustre l'évolution du point d'égalité subjective (PES) entre un groupe traité avec la composition 1 de l'invention et un groupe placebo.
La figure 2 illustre les « delta » (Δ) mesurés pour un groupe traité avec la composition 1 de l'invention et un groupe placebo.
La figure 3 illustre les changements individuels de delta (Δ) pour un groupe traité avec la composition 1 de l'invention et un groupe placebo.
La figure 4 illustre les courbes psychométriques obtenues dans le groupe traité avec la composition 1 de l'invention.

Les exemples suivants illustrent l'invention, ils ne la limitent en aucune façon.

### EXEMPLE 1

Les compositions préparées sont décrites dans les tableaux 1 à 3 ci-dessous.

**Tableau 1: Composition 1 sous forme de mousse**

| **Nom INCI ou dénomination chimique** | Fonction | % (m/m) |
|---|---|---|
| Pfaffia glomerata root juice | Actif vasodilatateur | 2,000 |
| Hyaluronate de sodium (PM : 1000 à 1400 KDa) | Agent hydratant | 0,075 |
| Hyaluronate de sodium (PM : 100 à 300 KDa) | Agent hydratant | 0,025 |
| Glycérol | Agent hydratant | 5,000 |
| Biosaccharide Gum-1 | Agent hydratant | 1,300 |
| Aloe vera (Aloe Barbadensis) | Agent hydratant | 0,050 |
| Nicotinamide | Agent hydratant | 0,100 |
| Carbomer | Agent viscosant | 0,200 |
| Polysorbate 20 | Agent moussant | 4,000 |
| Pentylène glycol | Agent de conservation | 2,000 |
| Ethyl héxyl glycérine | Agent de conservation | 0,250 |
| Sorbate de potassium | Agent de conservation | 0,200 |
| Parfum | Parfum | 0,050 |
| Hydroxyde de sodium | Ajusteur de pH | qs pH |
| Eau | Solvant aqueux | qs 100% |

**Tableau 2 : Composition 2 sous forme de mousse**

| **NOM INCI ou dénomination chimique** | Fonction | % (m/m) |
|---|---|---|
| Pfaffia glomerata root juice | Actif vasodilatateur | 2,000 |
| Hyaluronate de sodium (PM : 1000 à 1400 KDa) | Agent hydratant | 0,075 |
| Hyaluronate de sodium (PM : 100 à 300 KDa) | Agent hydratant | 0,025 |
| Glycérol | Agent hydratant | 3,000 |
| Biosaccharide Gum-1 | Agent hydratant | 1,300 |
| Aloe vera (Aloe Barbadensis) | Agent hydratant | 0,050 |
| Caprilic/Capric Triglycérides (nom INCI) | Agent hydratant | 6,000 |
| Alpha tocophérol | Antioxydant | 0,200 |
| Arachidyl alcohol & benehyl alcohol & arachidyl glucoside (Nom INCI) | Agent émulsionnant | 2,000 |
| Alcool cétylique | Agent émulsionnant | 2,000 |
| Decyl glucoside | Agent moussant | 3,000 |
| Isoamyl laurate | Agent moussant | 4,000 |
| Benzoate de sodium | Agent de conservation | 0,600 |
| Sorbate de potassium | Agent de conservation | 0,300 |
| Parfum | Parfum | 0,050 |
| Acide citrique | Ajusteur de pH | qs pH |
| Aqua | Solvant aqueux | qs 100% |

**Tableau 3 : Composition 3 sous forme de crème**

| **NOM INCI ou dénomination chimique** | Fonction | %(m/m) |
|---|---|---|
| Pfaffia glomerata root juice | Actif vasodilatateur | 2,000 |
| Hyaluronate de sodium (PM : 1000 à 1400 kDa) | Agent hydratant | 0,075 |
| Hyaluronate de sodium (PM : 100 à 300 KDa) | Agent hydratant | 0,025 |
| Glycérol | Agent hydratant | 6,000 |
| Aloe vera (Aloe barbadensis) | Agent hydratant | 0,050 |
| Caprilic/Capric Triglycérides | Agent hydratant | 1,500 |
| Caprylyl caprylate/caprate | Agent hydratant | 6,000 |
| Alpha tocophérol | Antioxydant | 0,200 |
| Carbomer | Agent de texture | 0,200 |
| PEG-6 stearate & glycol stearate & PEG-32 stearate | Agent émulsionnant | 7,000 |
| Apricot kernel oil PEG-6 esters | Agent émulsionnant | 2,000 |
| Ethyl héxyl glycérine | Agent de conservation | 0,500 |
| Benzoate de sodium | Agent de conservation | 0,125 |
| Sorbate de potassium | Agent de conservation | 0,125 |
| Eau | Solvant aqueux | qs 100% |

### EXEMPLE 2

L'étude décrite dans cet exemple évalue l'efficacité de la composition 1 (décrite dans l'exemple 1) sur la sensibilité tactile de sujets sains âgés de 60 à 75 ans après un mois d'application. La sensibilité tactile a été testée à l'aide de méthodes psychophysiques sur la base de la capacité des sujets à discriminer des textures synthétiques de différentes rugosités.

La plupart des travaux psychophysiques réalisés ces trente dernières années ont utilisé des tests susceptibles d'évaluer la sensibilité aux pressions et les capacités de discrimination tactile.

Le test le plus classique d'évaluation de la sensibilité aux pressions fines a été mis au point au début du XXe siècle lors des grandes heures de la psychophysique puis amélioré dans les années soixante (3). Il s'agit de monofilaments de nylon calibrés susceptibles d'appliquer une pression constante sur un espace de peau très réduit dès que le monofilament flanche. Ce test présente cependant de nombreux problèmes et aucun guide d'application n'a jamais été validé (4, 5).

Le test le plus classique d'évaluation de la discrimination tactile consiste en l'application simultanée des deux branches d'un compas à pointes sèches sur la peau et à diminuer l'écartement entre les branches jusqu'à ce que le sujet ne ressente qu'une seule pointe (Two-Point Gap Discrimination, TPGD) (3, 6, 7).

D'autres auteurs ont souvent utilisé des papiers de verre pour tester les capacités de discrimination tactile, comme par exemple récemment pour évaluer l'effet de la sensibilité tactile sur la préférence pour certains types de cosmétique (8). Les papiers de verre présentent l'avantage d'être faciles à utiliser et peu onéreux, mais ils présentent cependant de nombreux inconvénients. Les papiers les plus grossiers avec des grains de carbure de silicium de 425 µm sont abrasifs et attaquent la peau. Les papiers les plus fins utilisés avec des grains de 8,4 µm ne sont pas abrasifs mais ils présentent les mêmes inconvénients que les papiers les plus grossiers.

### PROTOCOLE

La sensibilité tactile a été évaluée dans la présente étude à l'aide d'un test original spécifiquement développé pour tester les compositions de l'invention.

Il a été demandé aux sujets de discriminer des plaques rainurées dont un seul paramètre variait : l'écartement inter-stries.

Plus particulièrement, les effets d'un mois d'application de la composition 1 ont été testé en double-aveugle contre placebo sur la sensibilité tactile de 42 sujets sains âgés de 60 à 75 ans. Les 42 sujets ont donc été répartis en deux groupes, l'un traité avec la composition 1 de l'invention, l'autre exposé à un placebo.

Le placebo ne comprend pas d'actif vasodilatateur et ne comprend pas d'acide hyaluronique et/ou ses sels. La composition du placebo est donnée dans le tableau 4 ci-dessous :

**Tableau 4 : composition placebo sous forme de mousse**

| Nom INCI ou dénomination chimique | Fonction | % (m/m) |
|---|---|---|
| Glycérol | Agent hydratant | 5,000 |
| Biosaccharide Gum-1 | Agent hydratant | 1,300 |
| Aloe vera (Aloe barbadensis) | Agent hydratant | 0,050 |
| Nicotinamide | Agent hydratant | 0,100 |
| Carbomer | Agent viscosant | 0,200 |
| Polysorbate 20 | Agent moussant | 4,000 |
| Pentylène glycol | Agent de conservation | 2,000 |
| Ethyl héxyl glycérine | Agent de conservation | 0,250 |
| Sorbate de potassium | Agent de conservation | 0,200 |
| Parfum | Parfum | 0,050 |
| Hydroxyde de sodium | Ajusteur de pH | qs pH |
| Eau | Solvant aqueux | qs 100% |

Les sujets devaient appliquer la crème sous forme de mousse (composition 1 ou placebo) deux fois par jour : une fois le matin (pas de lavage des mains durant l'heure suivant l'application) et une fois le soir au coucher. L'analyse de la sensibilité tactile a été conduite pour chaque sujet avant l'application de la crème (période dite « PRE ») et le 31^{ème} jour après très exactement (période dite « POST »).

Les caractéristiques hédoniques de la crème sous forme de mousse (agréabilité, texture, odeur ...), la facilité d'application et sa capacité de pénétration ont été évaluées par les sujets à l'aide d'échelles catégorielles rédigées en accord avec le commanditaire avec quatre notes possibles pour chaque item (tout à fait d'accord = 4, d'accord = 3, pas d'accord = 2, pas du tout d'accord = 1).

Il en est de même pour l'autoévaluation de la sensibilité tactile avant et après application de la crème. A mi-parcours et en fin de test, un indice de satisfaction générale a été calculé pour chaque sujet d'après la somme des notes quantitatives obtenues divisée par le nombre de questions.

L'évaluation de la sensibilité tactile a reposé sur une approche psychophysique du toucher sur la phalange la plus distale de l'index droit. Le choix de stimulus s'est porté sur une plaquette rainurée dont les stries sont d'écartement constant mais variable d'une plaquette à l'autre.

Les plaquettes ont été fabriquées par l'Institut FEMTO-ST (Franche-Comté Electronique Mécanique Thermique et Optique - Sciences et Technologies, UMR 6174, Besançon, Université de France-Conté et CNRS). Ces plaquettes sont en polycarbonate de dimension 50 mm x 80 mm, d'une épaisseur de 5 mm. Elles comportent des stries identiques, parallèles et régulièrement espacées. La striation de surface réalisée créée une strie entourée de deux bourrelets et ce sont davantage les bourrelets qui sont ressentis lorsqu'on touche les surfaces.

L'intérêt de ces textures imprimées en trois dimensions réside dans la maîtrise exacte de tous les paramètres physiques des stimuli utilisés et dans le fait de pouvoir faire varier un seul de ces paramètres : l'écartement inter-stries.

Une texture dite « référence » (« REF », codée 9 écartement 4,8 mm) a été définie. De part et d'autre de cette référence 4 plaquettes dites « TEST » ont été testées avec un pas de 0,2 mm et 1 plaquette avec un pas de 0.4 mm. En résumé ont été testées les plaquettes codées 6 / 7 / 7.5 / 8 / 8.5/9.5/10/10.5/11/12.

La méthode consiste à présenter les textures par paires REF/TEST ou l'inverse TEST/REF. De manière aléatoire, les huit plaquettes dont la largeur inter-stries varie de manière ascendante ou descendante par pas de 0,2 mm ont été présentées 15 fois autour de la référence et les plaquettes les plus éloignées de la référence (6 et 12) ont été présentées 6 fois. Au total, les sujets ont eu à évaluer 132 paires de plaquettes. Le temps moyen pour un test complet était d'environ 1h30 et les sujets disposaient d'une pause de cinq minutes tous les quarts d'heure.

Les sujets devaient garder les yeux fermés et étaient équipés d'un casque anti-bruit à filtre actif. Avec la phalange distale de l'index droit, ils devaient explorer chaque plaquette de haut en bas (les stries étant perpendiculaires par rapport au déplacement du doigt) à la vitesse de leur choix. Un seul passage était autorisé par plaque. Dans ces conditions, la plupart des sujets explorent les surfaces à une vitesse comprise entre 15 et 25 mm par seconde (9). Les sujets devaient alors répondre à la question suivante « quelle est la plaque dont les stries sont les plus écartées ? » en répondant 1 ou 2. Il s'agissait donc d'une tâche de choix forcé à deux alternatives.

A partir de ces réponses, les fonctions psychométriques peuvent être recherchées pour chaque sujet : il suffit d'exprimer le taux de discrimination en fonction de l'amplitude de l'écart inter-strie et de modéliser ces données par une fonction gaussienne cumulative. Deux paramètres sont alors extraits de ces courbes psychométriques :
- le point d'égalité subjective (PES), c'est-à-dire l'écart inter-stries pour lequel le participant répond au hasard, c'est-à-dire qu'il perçoit la texture test comme identique à la référence ; plus l'écart inter-stries va s'éloigner de ce PES, plus il va être aisé pour le participant de distinguer les textures « test » de la référence ;
- la pente de la courbe au PES qui traduit la capacité de discrimination du sujet ; plus la pente est raide, plus la différence d'espacement inter-strie pour être discriminée sera petite. On extrait ainsi le delta (Δ) qui correspond à l'intervalle d'intensités compris entre les abscisses correspondant au PES et à 75% de discrimination plus écartée. Plus la pente est raide, plus cet intervalle d'incertitude sera réduit.

Pour les données sociodémographiques de la population d'étude, un test de Mann-Whitney a été pratiqué. Il en a été de même pour les données issues des enquêtes de satisfaction réalisées à mi-parcours et après le mois d'application. Les échelles catégorielles construites de la façon suivante (tout à fait d'accord, d'accord, pas d'accord, pas du tout d'accord) ont été transformées en nombres entiers de 4 à 1, respectivement.

L'effet d'un mois d'application de la crème au sein de chaque groupe a été évalué avec un test T pour séries appariées. Le niveau de significativité a été fixé à *p* = 0,05. Pour toutes les différences significatives, un indice de puissance de l'effet (*effect size*) a été calculé selon les normes de Cohen (1992) (10), où *d* < 0,2 correspond à un effet faible, 0,3 < *d* < 0,5 correspond à un effet moyen et *d* > 0,5 à un effet puissant. Le *d* de Cohen évalue la différence entre les moyennes de chaque groupe selon leurs propres écart-types. Ainsi, un effet puissant signifie qu'un groupe se distingue bien de l'autre non pas grâce à une ou deux valeurs extrêmes qui amènent à une différence significative, mais bien parce que la majorité des observations individuelles sont différentes.

### RESULTATS ET CONCLUSIONS

### Mesures psychométriques de la sensibilité tactile - Comparaisons PRE / POST

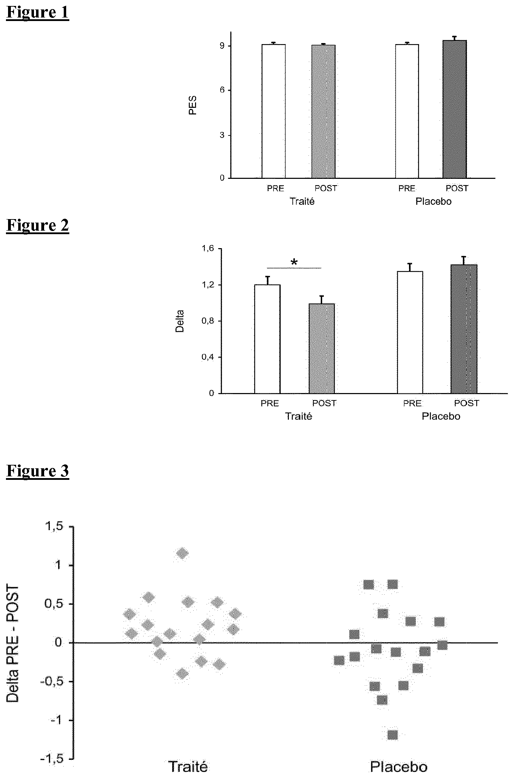

Lors de la première séance d'évaluation de la sensibilité tactile, le point d'égalité subjective (PES) ne variait pas entre les groupes traité (9.3 ± 0.4) et placebo (9.2 ± 0.3) (voir Figure 1, Z = 1.19, Z limite = 1.96, Z = 1.1, *p* = 0.23).

Le point d'égalité subjective est resté tout aussi constant après un mois d'application de crème, que ce soit dans le groupe traité (9.2 ± 0.3) (T = 0.98, T limite = 2.12, p = 0.34) ou dans le groupe placebo (9.4 ± 0.5) (T = 1.67, T limite = 2.12, *p* = 0.11).

Nous pouvons donc en conclure que les sujets des deux groupes ressentaient bien la plaquette référence de la même façon avant l'application de la crème et, qu'au sein de chaque groupe, le mois d'application de la composition 1 de l'invention n'a pas eu d'effet sur la perception de cette plaquette référence.

En ce qui concerne la pente de la courbe au PES, l'indice qui traduit la capacité de discrimination des sujets, le delta (Δ) était similaire entre les groupes traité et placebo lors du premier test en période PRE, avant le mois d'application de la crème. Ces résultats sont reportés sur la Figure 2 (Z = 1.12, Z limite = 1.96, p = 0.26).

En revanche, dans le groupe traité avec la composition 1 de l'invention, le delta (Δ) est significativement diminué après un mois d'application de la crème (voir fig. 2, T = 2.41, T limite = 2.12, p = 0.03). Cet effet significatif est associé à un *d* de Cohen égal à 0.57, ce qui correspond à un effet puissant. Dans le groupe placebo, le delta demeure inchangé (*p* = 0.54). Ainsi, les sujets du groupe actif sont passés d'une incertitude moyenne de discrimination de 0.48 mm à 0.40 mm après un mois d'application, soit une amélioration de la discrimination tactile de 83 µm, en moyenne.

La différence entre les deltas mesurés en PRE et POST application a été calculée à l'échelle individuelle. Ainsi, une valeur positive correspond à une amélioration de la sensibilité tactile, une valeur autour de zéro à aucun effet et une valeur négative à une dégradation de la sensibilité tactile. Les résultats sont reportés dans la Figure 3.

Dans le groupe traité, 12 sujets ont eu un delta augmenté, et 4 un delta diminué. Dans le groupe placebo, 6 sujets ont eu un delta augmenté et 11 un delta diminué.

Ainsi, l'amélioration de la discrimination est bien plus fréquente chez les sujets du groupe traité avec la composition 1 de l'invention.

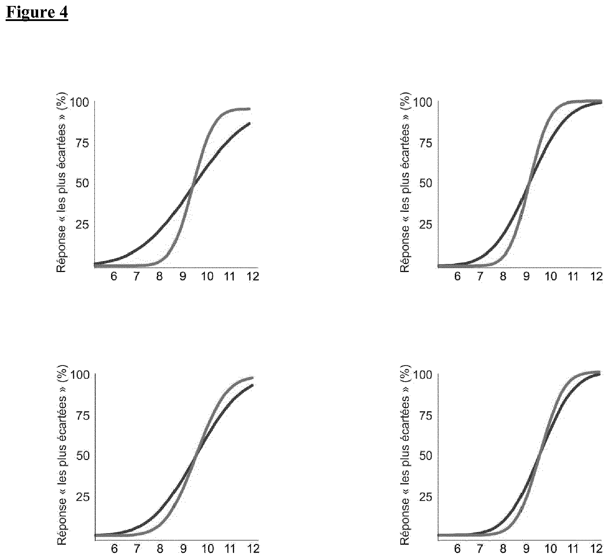

La Figure 4 illustre les courbes psychométriques obtenues après traitement statistique chez quatre sujets du groupe traité. Il apparaît très clairement que l'application de la composition 1 de l'invention pendant un mois s'accompagne d'une augmentation de la pente, c'est-à-dire d'une amélioration des capacités de discrimination tactile.

### Conclusion :

L'application deux fois par jour pendant un mois de la composition 1 de l'invention augmente de façon significative la discrimination fine de textures synthétiques. Cet effet est confirmé chez la plupart des sujets exposés au produit actif.

### Références bibliographiques

1. Da Silva LA, Lin SM, Teixeira MJ, de Siqueira JTT, Jacob Filho W, de Siqueira SRDT. Sensorial differences according to sex and ages. Oral Dis. Avr 2014; 20(3):103-110.
2. Stevens JC, Choo KK. Spatial acuity of the body surface over the life span. Somatosens. Mot Res. 1996; 13(2):153-66.
3. Weinstein S. Fifty years of somatosensory research: from the Semmes-Weinstein monofilaments to the Weinstein Enhanced Sensory Test. J Hand Ther Off J Am Soc Hand Ther. Mars 1993; 6(1):11-22; discussion 50.
4. Massy-Westropp N. The effects of normal human variability and hand activity on sensory testing with the full Semmes-Weinstein monofilaments kit. J Hand Ther Off J Am Soc Hand Ther. mars 2002;15(1):48-52.
5. Levin S, Pearsall G, Ruderman RJ. Von Frey's method of measuring pressure sensibility in the hand: an engineering analysis of the Weinstein-Semmes pressure aesthesiometer. J Hand Surg. Mai 1978; 3(3):211-6.
6. Johansson RS, Vallbo AB. Spatial properties of the population of mechanoreceptive units in the glabrous skin of the human hand. Brain Res. 24 févr 1980; 184(2):353-66.
7. Aimonetti J-M, Radovanovic S. Somesthesis. In: Handbook of physics in medicine and biology. Taylor & Francis Group. New York: Robert Slinter; 2010; p. 1-22.
8. Trautmann M, Wendel V, Prinz D, Primmel B, Willging G, Nagorsen E, et al. Not only age but also tactile perception influences the preference for cosmetic creams applied to the forearm. Int J Cosmet Sci. 16 nov 2016.
9. Vega-Bermudez F, Johnson KO, Hsiao SS. Human tactile pattern récognition: active versus passive touch, velocity effects, and patterns of confusion. J Neurophysiol. Mars 1991; 65(3):531-46.
10. Cohen J. A power primer. Psychol Bull., 1992;112(1):155-9.

## Revendications

1. Actif vasodilatateur choisi dans le groupe comprenant le ginseng brésilien (*Pfaffia glomerata),* le ginseng sibérien (*Eleutherococcus senticosus),* le ginseng rouge de Corée (*Panax ginseng*), le ginseng américain (*Panax quinquefolius*)*,* la maca (*Lepidium meyenii*)*,* le guarana (*Paullinia cupana*), la caféine, la théine, le muira puama *(Ptychopetalum olacoides),* l'abricotier d'argent (*Ginkgo biloba*), le yohimbe (*Pausinystalia johimbe*), le tribulus *(Tribulus terrestris),* le gingembre (*Zingiber officinale*), la cannelle (*Cinnamomum verum*), le céleri (*Apium graveolens*) et leurs mélanges, et de préférence le ginseng brésilien,
en association avec de l'acide hyaluronique et/ou ses sels,
et éventuellement avec un agent hydratant choisi dans le groupe comprenant des héparanes sulfates, du glycérol, un polysaccharide anionique (Biosaccharide gum-1), du xylitol, du fructose, du collagène, un ester triple de glycérol et des acides caprylique et caprique (nom INCI : caprilic/capric triglycérides), du caprylyl caprylate/caprate (nom INCI), de la nicotinamide, de l'Aloe vera (*Aloe barbadensis*) et leurs mélanges, et de préférence des héparanes sulfates,
pour une utilisation dans la prévention et/ou le traitement des troubles de la somesthésie, et plus particulièrement de l'hypoesthésie.

2. Actif vasodilatateur pour une utilisation selon la revendication 1, dans laquelle le Ginseng brésilien se trouve sous forme de jus de racine.

3. Actif vasodilatateur pour une utilisation selon la revendication 1, dans laquelle l'acide hyaluronique est un mélange de deux acides hyaluroniques, de préférence sous forme de leurs sels, présentant respectivement un poids moléculaire allant de 100 à 300 KDa et allant de 1000 à 1400 KDa.

4. Actif vasodilatateur pour une utilisation selon l'une quelconque des revendications 1 à 3, chez des sujets âgés d'au moins 35 ans, de préférence d'au moins 55 ans, et plus préférentiellement encore d'au moins 60 ans.

5. Composition topique comprenant :
- un actif vasodilatateur choisi dans le groupe comprenant le ginseng brésilien (*Pfaffia glomerata),* le ginseng sibérien (*Eleutherococcus senticosus*), le ginseng rouge de Corée (*Panax ginseng*), le ginseng américain (*Panax quinquefolius*), la maca (*Lepidium meyenii*)*,* le guarana (*Paullinia cupana*), la caféine, la théine, le muira puama (*Ptychopetalum olacoides*), l'abricotier d'argent (*Ginkgo biloba*), le yohimbe (*Pausinystalia johimbe*), le tribulus *(Tribulus terrestris),* le gingembre (*Zingiber officinale*), la cannelle (*Cinnamomum verum*), le céleri (*Apium graveolens*) et leurs mélanges, et de préférence le ginseng brésilien,
- de l'acide hyaluronique et/ou ses sels,
- éventuellement un agent hydratant choisi dans le groupe comprenant des héparanes sulfates, du glycérol, un polysaccharide anionique (Biosaccharide gum-1), du xylitol, du fructose, du collagène, un ester triple de glycérol et des acides caprylique et caprique (nom INCI : caprilic/capric triglycérides), du caprylyl caprylate/caprate (= nom INCI), de la nicotinamide, de l'Aloe vera (*Aloe Barbadensis*) et leurs mélanges, et de préférence des héparanes sulfates,
- de l'eau,
- éventuellement un agent de conservation choisi dans le groupe comprenant le pentylène glycol, le sorbate de potassium, le phénoxyéthanol, l'éthyl héxyl la glycérine, le benzoate de sodium, le gluconolactone, le capryloyl glycine, l'octopirox, l'alcool phényléthylique et leurs mélanges,
pour une utilisation dans la prévention et/ou le traitement des troubles de la somesthésie, et plus particulièrement de l'hypoesthésie.

6. Composition topique pour une utilisation selon la revendication 5, dans laquelle :
- l'actif vasodilatateur est présent en une quantité allant de 1 à 10%, de préférence de 1,5 à 5%,
- l'acide hyaluronique et/ou ses sels est présent en une quantité allant de 0,01 à 1%, de préférence de 0,05 à 0,5%,
- l'agent hydratant est présent en une quantité allant de 3 à 30%, de préférence de 5 à 15%,
- l'eau est présente en une quantité allant de 60 à 90%, de préférence de 70 à 85%,
- l'agent de conservation est présent en une quantité allant de 0,1 à 5%, de préférence de 0 ,5 à 2,5%,
les pourcentages étant exprimés en poids par rapport au poids total de la composition.

7. Composition topique pour une utilisation selon la revendication 5 ou la revendication 6, pour une application sur les mains et/ou les pieds.

8. Composition topique pour une utilisation selon la revendication 5 ou la revendication 6, pour une application sur les muqueuses buccales, nasales et/ou vaginales.

9. Composition topique pour une utilisation selon l'une quelconque des revendications 5 à 8, chez des sujets âgés d'au moins 35 ans, de préférence d'au moins 55 ans, et plus préférentiellement encore d'au moins 60 ans.
